# EUROPEAN PATENT SPECIFICATION

(11) **EP 2 232 236 B1**
(45) Date of publication and mention of the grant of the patent: **18.12.2019**
(21) Application number: 08864392.9
(22) Date of filing: 19.12.2008
(51) Int. Cl.: G01N 21/3577, G01N 21/359, G01N 21/64, G01N 33/30, G01N 21/85, G01N 21/94

(54) **MONITORING LUBRICANT OIL CONDITION AND/OR QUALITY, ON-LINE OR AT-LINE, BASED ON CHEMOMETRIC DATA ANALYSIS OF FLOURESCENCE AND/OR NEAR INFRARED SPECTRA**
ÜBERWACHUNG DES ZUSTANDS UND/ODER DER QUALITÄT VON SCHMIERÖL ON-LINE ODER AT-LINE AUF DER GRUNDLAGE CHEMOMETRISCHER DATENANALYSE VON FLUORESZENZ- UND/ODER NAHINFRAROTSPEKTREN
SURVEILLANCE D'UNE CONDITION ET/OU D'UNE QUALITÉ D'HUILE LUBRIFIANTE, EN LIGNE OU DE LIGNE, SUR LA BASE D'UNE ANALYSE DE DONNÉES CHIMIOMÉTRIQUES DE FLUORESCENCE ET/OU DE SPECTRES PROCHES INFRAROUGES

(30) Priority: 21.12.2007 DK 200701877; 02.01.2008 US 18512
(43) Date of publication of application: 29.09.2010
(73) Proprietor: Medico Kemiske Laboratorium ApS, 2950 Vedbaek (DK)
(72) Inventor: OLSEN, Ole, DK-2920 Charlottenlund (DK); KAMPMANN, Kristoffer, DK-2100 Copenhagen (DK)
(74) Representative: Høiberg P/S
(86) International application number: PCT/DK2008/050329
(87) International publication number: WO 2009/080049

(56) References cited:
- EP-A1- 1 818 666
- US-A- 5 223 715
- US-A- 5 349 189
- US-A- 5 424 959
- US-A1- 2003 056 581
- US-A1- 2004 084 623
- US-A1- 2004 106 204
- US-B1- 6 455 850
- ABBAS O ET AL: "Application of chemometric methods to synchronous UV fluorescence spectra of petroleum oils" FUEL, IPC SCIENCE AND TECHNOLOGY PRESS, GUILDFORD, GB, vol. 85, no. 17-18, 1 December 2006 (2006-12-01), pages 2653-2661, XP025235882 ISSN: 0016-2361 [retrieved on 2006-12-01]
- CANECA A R ET AL: "Assessment of infrared spectroscopy and multivariate techniques for monitoring the service condition of diesel-engine lubricating oils" TALANTA, ELSEVIER, AMSTERDAM, NL, vol. 70, no. 2, 15 September 2006 (2006-09-15), pages 344-352, XP025000781 ISSN: 0039-9140 [retrieved on 2006-09-15]

## Description

The present invention relates to a method of training a system for and a method for characterising a lubricant oil sample.

This makes it possible to implement a device and a method that will work both on-line, by measuring directly in an oil stream, and at-line, where a collected sample is measured in an instrument placed close to the place of sampling.

### Background

US 2007/0187617 discloses a method for monitoring the oxidation of oil samples by exposing the sample to ultraviolet irradiation and detecting in 2 narrow wavelength ranges the emitted fluorescence. The ratio between the intensity of the fluorescence emitted in the different wavelength ranges changes as a result of the oxidation of the oil, and by tracking the value of this ratio the quality of the oil can be monitored. This method only derives information from a small fraction of the wavelength range emitted from the oil sample. Furthermore, this method has limited used for analyzing unknown samples.

Other prior art techniques for detecting the content of water in oil rely on the scattering of light on water. Such techniques are sensitive to the scattering of light on for example air bubbles or other impurities, and do hence not provide a reliable measure of the water content in the sample.

US 2004/084623 discloses a method for providing rapid on-line analyses of chemical compositions such as chemical process streams, utilizing near-infrared (NIR) spectroscopy in combination with chemometrics.

In Fuel 85 (2006) 2653, "Application of chemometric methods to synchronous UV fluorescence spectra of petroleum oils" by Abbas et al., a novel approach for identification and discrimination of oils by chemometric treatment of synchronous ultraviolet fluorescence (SUVF) spectra is described.

In Talanta 70 (2006) 344, "Assessment of infrared spectroscopy and multivariate techniques for monitoring the service condition of diesel-engine lubricating oils" by Caneca et al., two methodologies for monitoring the service condition of diesel-engine lubricating oils on the basis of infrared spectra are presented.

US 2003/056581 discloses a method for determining the concentration of a constituent in a fluid by directing a beam of light into the fluid and sensing the intensity of components of the light emerging from the fluid at various wavelengths.

US 5,223,715 discloses a process for obtaining spectral information and for quantifying the physical properties of a sample, based on polychromatic light transmitted through the sample.

US 5,349,189 discloses a process for determining component concentration in a mixture, where the components are paraffins, isoparaffins, aromatics, naphthenes, or olefins, where the process comprises measuring the absorbance in near infrared.

### Summary

It is an object of the present invention to provide a reliable method capable of predicting quality parameters of lubricant oil samples with unknown properties and/or classifying said lubricant oil samples in a system not requiring any drying, enrichment or concentration of the lubricant oil sample before determining the quality parameter and/or class, to which the lubricant oil sample belongs.

This is possible by subjecting the lubricant oil sample to fluorescent and/or near infrared (NIR) spectroscopy or variants thereof, and analyzing the detected light transmitted through and/or emitted from and/or reflected from the lubricant oil sample, whereby lubricant oil samples may be classified. This classification and monitoring can be performed with a minimum of lubricant oil sample preparation if any at all.

Thus in a first aspect the present invention relates to a method of training a system for characterising a lubricant oil sample, said lubricant oil sample being obtained directly from an oil engine, a transmission, or any other lubricant oil using unit and said lubricant oil sample being subjected to the method without any changes in concentration, said method comprising the steps of
a) providing a batch of lubricant oil samples having a predetermined content for training the system to recognise at least one characteristic of the sample,
b) exposing a lubricant oil sample from said batch to electromagnetic radiation within a predetermined range of wavelengths,
c) monitoring the electromagnetic radiation emitted from the lubricant oil sample from said batch at an exposing range from 200 nm to 800 nm, and/or monitoring the electromagnetic radiation transmitted through and/or reflected from the lubricant oil sample from said batch at an exposing range from 750 nm to 3000 nm, in order to determine at least one physical spectroscopic parameter,
d) repeating step b) to c) until the at least one physical spectroscopic parameter of all lubricant oil samples of said batch have been determined,
e) performing data processing of the obtained physical spectroscopic parameters thereby obtaining data variables,
f) performing a multivariate data analysis of the obtained data variables obtaining model parameters describing the variation of the data variables,
g) obtaining characterisation information related to each lubricant oil sample from said batch.

In another aspect, the invention relates to a method for characterising a lubricant oil sample, said lubricant oil sample being obtained directly from an oil engine, a transmission, or any other lubricant oil using unit and said lubricant oil sample being
subjected to the method without any changes in concentration, said method comprising the steps of:
a) obtaining the lubricant oil sample,
b) exposing the lubricant oil sample to electromagnetic radiation within one or both range(s) of wavelengths from 200 nm to 800 nm and/or from 750 nm to 3000 nm,
c) determining at least one physical spectroscopic parameter of electromagnetic radiation emitted from the lubricant oil sample at an exposing range from 200 nm to 800 nm, and/or determining at least one physical spectroscopic parameter of electromagnetic radiation transmitted and/or reflected from the lubricant oil sample at an exposing range from 750 nm to 3000 nm,
d) performing data processing of the obtained at least one physical spectroscopic parameter obtaining at least one data variable,
e) storing the obtained at least one data variable,
f) providing at least one latent variable from data variables of the sample,
g) obtaining at least one latent variable from a trained prediction and/or classification system,
h) correlating latent data variables from the sample with latent variables of the trained prediction and classification system, to obtain quantitative predictions and/or quality predictions and/or at least one characterisation class of the sample, and
i) displaying the quantitative predictions and/or quality predictions and/or at least one characterisation class of the sample.

The method is preferably carried out in a system trained according to the present invention.

### Description of Drawings

- Figure 1:: NIR spectra from oil samples
- Figure 2:: Laboratory value versus the value predicted by the chemometric model and NIR spectroscop for water
- Figure 3:: Laboratory value versus the value predicted by the chemometric model and NIR spectroscop for viscosity at 40 degrees
- Figure 4:: Laboratory value versus the value predicted by the chemometric model and NIR spectroscop for TAN,
- Figure 5:: Laboratory value versus the value predicted by the chemometric model and NIR spectroscop for TBN
- Figure 6:: Laboratory value versus the value predicted by the chemometric model and NIR spectroscop for Silicon
- Figure 7:: Emission spectra at 230 nm excitation for several oil samples.
- Figure 8:: Classification of new and used samples.
- Figure 9:: Extended Canonical Variates for new and old oil samples
- Figure 10:: Laboratory value versus the value predicted by the chemometric model and NIR spectroscop for viscosity. PLS model on Main engine oil in use (group 1) and Main engine new oil (group 2)for KV100. PLSC is 6. Data is preprocessed with Savitzky-Golay (w =9, O=2, d=1).
- Figure 11:: Laboratory value versus the value predicted by the chemometric model and NIR spectroscop for density. PLS model on Main engine oil in use and Main engine new oil (group 1 and 2) for Density. PLSC is 7. Data is preprocessed with Savitzky-Golay (w =9, O=2, d=1).
- Figure 12:: Laboratory value versus the value predicted by the chemometric model and NIR spectroscop for TAN. PLS model on Main engine oil in use and Main engine new oil (group 1 and 2) for TAN177. PLSC is 3. The data is preprocessed with Savitzky-Golay (w =9, O=2, d=1).
- Figure 13:: Laboratory value versus the value predicted by the chemometric model and NIR spectroscop for TBN. PLS model on Main engine oil in use and Main engine new oil (group 1 and 2) for TBN PLSC is 7. Data is preprocessed with Savitzky-Golay (w =9, O=2, d=1).
- Figure 14:: Laboratory value versus the value predicted by the chemometric model and NIR spectroscop for sulfur. PLS model on Main engine oil in use and Main engine new oil (group 1 and 2) for S. PLSC is 1. Data is pre-processed with Savitzky-Golay (w =9, O=2, d=1).
- Figure 15:: Laboratory value versus the value predicted by the chemometric model and NIR spectroscop for phosphor. PLS model on Main engine oil in use and Main engine new oil (group 1 and 2) for P. PLSC is 12. Data is preprocessed with Savitzky-Golay (w =9, O=2, d=1).
- Figure 16:: Laboratory value versus the value predicted by the chemometric model and NIR spectroscop for vanadium. PLS model on Main engine oil in use and Main engine new oil (group 1 and 2) for V. PLSC is 7. Data is preprocessed with Savitzky-Golay (w =9, O=2, d=1).
- Figure 17:: Laboratory value versus the value predicted by the chemometric model and NIR spectroscop for nickel. PLS model on Main engine oil in use and Main engine new oil (group 1 and 2) for Ni. PLSC is 10. Data is preprocessed with Savitzky-Golay (w =9, O=2, d=1).
- Figure 18:: Laboratory value versus the value predicted by the chemometric model and NIR spectroscop for magnesium. PLS model on Main engine oil in use and Main engine new oil (group 1 and 2) for Mg. PLSC is 8. Data is preprocessed with Savitzky-Golay (w =9, O=2, d=1).
- Figure 19:: Laboratory value versus the value predicted by the chemometric model and NIR spectroscop for calcium. PLS model on Main engine new oil and Main engine oil in use (group 1 and 2) for Ca. PLSC is 5. Data is preprocessed with Savitzky-Golay (w =9, O=2, d=1).

### Detailed description of the invention

The invention relates to quantitative prediction of physical and/or chemical values or qualitative classification of lubricant oils sample status based on spectroscopic parameters obtained from near infrared and/or luminescence spectroscopy. Fluorescence spectroscopy is used as an example of luminescence spectroscopy in the present invention. However, as described below other physical parameters may be used in the prediction and/or classification. Thus, throughout the description the term fluorescence is used as an equivalent of any luminescence type and is to be interpreted as such, unless inappropriate in specific embodiments.

Fluorescence spectroscopy is an extremely sensitive analytical tool and near infrared spectroscopy is a very precise analytical tool. The data obtained from both types of spectroscopic analyses can be considered a finger-print of the sample. Each sample gives rise to a unique spectroscopic set of physical parameters, and as is described in the present invention it is possible to form the finger-prints to predict the value of physical-chemical components of the oil, such as water, viscosity (at different temperatures), Total Base Number (TBN), Total Acid Number (TAN), carbon residue, density, flash and fire points, pour point, sulphated ash, score values in various performance tests, and the content of Particles, Silicon, Sodium, Boron, Iron, Aluminum, Chromium, Molybdenum, Copper, Lead, Tin, Nickel, Titanium, Silver, Phosphorus, Zinc, Calcium, Barium, Magnesium, and Sulfur.

The fluorescence and near infrared spectroscopic fingerprint can be used either alone or in a combination to obtain synergy in the prediction models. Furthermore, as also described by the present invention, when analysing the spectroscopic data, it has become possible to classify lubricant oil samples into two or more classes based on the spectra, if there is any systematic difference between the lubricant oil samples. The difference between the lubricant oil samples will mostly not relate to a single component, or a few components of the lubricant oil sample, but rather to a combination of a wide variety of components.

This combination exhibits a pattern so complex that it is detectable by the multivariate analyses only. Examples of the classification information provided by the present invention may be any information regarding sample condition, such as information regarding presence/absence of specific components, determination if the lubricant oil is used/not used (can also be quantified), determination of which type of engine the
lubricant oil is used in. The oil can be a fuel.

Thus, according to the evaluation of the spectroscopic parameters it is possible to obtain more information about a lubricant oil sample, than it is when evaluating the various chemical components in the lubricant oil sample individually, i.e. it is possible to obtain inter-component information. Furthermore, there is no need to know the exact composition of components in the lubricant oil sample, as it is either the fluorescence or the near infrared absorption spectra or the combination of these finger-prints that contain the relevant information. If so desired, in a specific application, it may be possible to give a chemical characterisation of the information used by the classification system.

For one lubricant oil sample normally over 100 data variables are obtained. The amount of data variables increases by the number of lubricant oil samples used but the number of data variables is constant for each lubricant oil sample. It is common practise to discard most of the spectroscopic information and use but a few selective or semi-selective spectroscopic parameters but the present invention makes use of all available information.

An embodiment of the invention relates to a method of training a prediction system for quantifying multiple chemical and/or physical parameters in a lubricant oil sample. It is the purpose of the training that a system is obtained, said system holding enough information to be used for quantitative prediction of multiple chemical and/or physical parameters in an unknown lubricant oil sample. By the term unknown is meant a lubricant oil for which no characterisation information is known and only the near infrared and/or fluorescence spectrum is known. The prediction as such is only based on a model and the measured spectroscopic fingerprint(s).

Another embodiment of the invention relates to a method of training a classification system for characterising a lubricant oil sample. It is the purpose of the training that a classification system is obtained, said system holding enough information to be used for characterising an un-classified and unknown lubricant oil sample into one of the classes of the classification system. By the term unknown is meant a lubricant oil sample for which no characterisation information is known.

It is also the purpose of the training of both the prediction and the classification systems that this training incorporates a validation that substantiates how well prediction and/or classification can be performed on specific lubricant oil samples in the future as well as improving the validation performance over time.

The electromagnetic radiation exposing the lubricant oil sample comprises monitoring
electromagnetic radiation and/or excitation light.

### Samples

The lubricant oil sample may be any lubricant oil sample suitable for spectroscopic analyses. It is an object of the present invention to acquire the necessary information from the lubricant oil sample using as few pre-treatments as possible, preferably without any pre-treatments as such.

Accordingly, the lubricant oil sample is transferred directly as is to be subjected to spectroscopic analysis, in order to obtain data relating to untreated lubricant oil samples.

The lubricant oil sample is subjected to spectroscopic analyses without drying, and without any other changes in concentration. The lubricant oil sample may be arranged in a sample compartment being closed or open before exposing the lubricant oil sample to light.

Furthermore, it is possible to use a lubricant oil sample positioned on-line or in-line, i.e. in a process stream.

### FLUORESCENCE SPECTROSCOPY

### Excitation light

The physical parameters may in principle be obtained for a wide variety of excitation light wavelengths. The wavelengths are selected to be within a range from 200 nm to 800 nm, and are preferably selected to be within the range from 200 nm to 600 nm.

Normally several wavelengths are used, such as from 2 to 100 wavelengths, for instance 2-30, such as 2-10, for instance 2-6 wavelengths in order to describe an excitation-emission matrix optimally. Sets of wavelengths may be chosen so that each wavelength differs from the other by at least 0.1 nm, such as 0.5 nm, for instance at least 1 nm, such as 5 nm, for instance at least 10 nm, such as 50 nm, for instance at least 100 nm, such as 150 nm, for instance at least 250 nm, such as 500 nm, for instance at least 600 nm, such as 700 nm, and at most 750 nm.

Accordingly, more than 2 excitation light wave lengths are selected such as 4, 6, 8, 10, or more. The excitation light of each wavelength may be used simultaneously or sequentially. In a preferred embodiment 4 wavelengths are selected, such as excitation light having a wavelength of 230 nm, 240 nm, 290 nm, and 340 nm. Each sample is then subjected to excitation light of each wavelength.

The predetermined excitation light wavelength(s) is provided by use of conventional light sources combined with a dispersive element such as a monochromator and/or optical-acoustical wavelength filters and/or conventional filters as is known to a person skilled in fluorescence spectroscopy.

### Measured physical emission parameters

Mostly the physical parameters to be determined in order to perform a data analysis are the intensities as a function of excitation wavelength and/or the emission wavelength. However the measurement instrument measuring the intensity may provide other information such as fluorescence lifetime, phosphorescence intensity, as well as phosphorescence lifetime, depolarisation, quantum yield, phase-resolved emission, and circular depolarization.

By the term "physical emission parameters" is meant any physical parameter capable of providing a luminescence related property of the sample.

Fluorescence intensity is easily measured at room temperature, and may therefore be chosen for many of the samples. Furthermore, a great number of organic natural products are known to be fluorescent. Phosphorescence may however also be performed at room temperature.

Luminescence lifetime in general, as well as phosphorescence lifetimes are defined as the time required for the emission intensity to drop to 1/e of its initial value.

When using phase resolved fluorescence spectroscopy it is possible to suppress Raman and scattered light, leading to very good results for multicomponent systems.

In luminescence polarisation measurements, spectra are obtained by scanning excitation spectra and measuring intensity with polarizers transmitting in the planes parallel and perpendicular to the polarisation plane the of exciting light. The degree of polarization or anisotropy may be calculated from the difference of the two measurements to the sum of the two measurements.

### Emission

The emitted light is dispersed or filtered and detected by any suitable detector, such as a scanning camera, a photomultiplier, a diode array, a CCD or a CMOS, all in principle being viewed as two-dimensional array of several thousand or more detectors. The intensity of the light is detected on each detector. In some embodiments one-dimensional detectors may be used as well such as a photomultiplier.

The determination of the various physical parameters is done by conventional equipment known to the person skilled in the art.

In fluorescence spectroscopy emission light intensities at different wavelengths are recorded for each excitation light wavelength. Preferably the emission light is sampled with 1 nm intervals. Thereby a matrix of excitation-emission data is obtainable for each lubricant oil sample. The spectral distribution of light emitted from the lubricant oil sample is ranging from 200 nm to 800 nm.

The emitted light from the lubricant oil samples may be focused onto the detectors by means of conventional focusing systems, as well as passing through diaphragms and mirrors.

### Processing

The detectors are preferably coupled to a computer for further processing of the data. The physical parameters measured or determined by the detector are processed to a form suitable for the further mathematical calculations. This is done by allocating data variables to each physical parameter determined, thus obtaining data variables related to the physical parameters,

The physical parameters determined are often subjected to a data analysis through the data variables, such as a one-way matrix of spectral information, a two-way matrix of spectral information, a three-way matrix of spectral information, a four-way matrix of spectral information or, a five-way or higher order matrices of spectral information.

### Characterizing the monitoring NIR radiation

The NIR absorption spectrum may in principle be obtained for a wide variety of radiation wavelengths. The wavelengths are selected to be within the range of from 750 nm to 3000 nm, preferably from 900 to 2500 nm.

Normally several wavelengths are used, such as from 2 to 5000 wavelengths. Sets of wavelength may be chosen so that each wavelength differs from the other by at least 0.1 nm, such as 0.5 nm, for instance at least 1 nm, such as 5 nm, for instance at least 10 nm, such as 50 nm, for instance at least 100 nm, such as 150 nm.

The absorption at each wavelength may be used simultaneously or sequentially. In a preferred embodiment 4 wavelengths are selected, such as monitoring light having a wavelength of 1300 nm, 1500 nm, 1700 nm, and 1900 nm. The monitoring may also use the wavelengths 2870nm and 3050nm where the absorption of electromagnetic radiation in water is strong. Each lubricant oil sample is then subjected to monitoring radiation of each wavelength.

The predetermined monitoring radiation wavelength(s) are provided by use of conventional light sources, dispersive elements and/or filters as is known to a person skilled in near infrared spectroscopy.

Mostly the physical NIR parameters to be determined in order to perform a data analysis are the absorbance and/or transmission values as a function of wavelength.

Near infrared signals are easily measured at room temperature, and may therefore be chosen for many of the lubricant oil samples. Furthermore, a great number of organic natural products are known to absorb in the near infrared region.

### Near infrared light detection

The transmitted NIR radiation is detected by any suitable detector, such as a semiconductor (e.g. PbS), photo voltaic elements (e.g. InAs, InSb), or a CCD. The intensity of the light is detected on each detector. In some embodiments one-dimensional detectors may be used as well such as a photomultiplier.

In near infrared spectroscopy, absorbance and/or transmission values are recorded for each excitation light wavelength. Preferably the detection of light is sampled with 1 nm intervals.

The signals can be recorded also by fibre optic probes, transmission probes, transmission probes for process applications, or reflection/backscattering probes.

### Processing fluorescence and NIR data

The near infrared and/or the fluorescence detectors are preferably coupled to a computer for further processing of the data. The physical parameters measured or determined by the detector(s) are processed to a form suitable for the further mathematical calculations. This is done by allocating data variables to each physical parameter determined, thus obtaining data variables related to the physical parameters,

The physical parameters determined are often subjected to a data analysis through the data variables, such as a one-way matrix of spectral information, a two-way matrix of spectral information, a three-way matrix of spectral information, a four-way matrix of spectral information or, a five-way or higher order matrices of spectral information or mixtures of these data structures.

### Characterisation information for lubricant oil samples

To obtain a system for quantification of one or several lubricant oil quality parameters or determination of a specific condition in the lubricant oil sample it is of importance that the data relating to the spectra obtained are correlated to characterisation information regarding the same lubricant oil sample. The characterisation information relates to the values (e.g. concentrations) of physical-chemical components in the lubricant oil or the classes characterizing the lubricant oil samples. The characterisation information may give information of both qualitative and quantitative information.

The characterisation information must be correlated to the spectral information obtained from the lubricant oil sample, in order to obtain the trained system ready for testing unknown lubricant oil samples.

### Validity

Each lubricant oil sample is subjected to fluorescence and/or near infrared spectroscopy before the data analysis is performed in the training of the prediction and/or classification system. The lubricant oil sample may be one sample from a lubricant oil sample, or several samples from the same lubricant oil sample or process, each sample obtained at a different time interval or from different process streams or from different instruments.

Depending on the predictions to be performed or the classes to be identified when training the classification system it is of importance to train the system with a sufficient number of lubricant oil samples. The determination of the sufficient number of lubricant oil samples is primarily determined by the number of expected latent variables. It is preferred that the ratio of number of training lubricant oil samples to the expected number of latent variables is at least 5:1, preferably at least 10:1. More preferred the ratio is 50:1, and even more preferred 100:1. The more training lubricant oil samples, the more reliable a system. Training is a continual improvement of the system and any lubricant oil sample is also a training sample however to a decreasing degree over time.

The lubricant oil samples being classified in each class are preferably a representative group of lubricant oil samples to allow the most reliable classification, wherein representative is meant to mean exhibiting all variations influencing said prediction and classification. These variables can for example be oil type, process type, engine type etc.

### Mathematics

A central aspect of the invention is the performance of a multivariate analysis, whereby the data variables relating to the physical spectroscopic parameters are evaluated. The latent variables being weighted averages of the data variables are obtained. The latent variables describe the variation of the data variables. Thereby, the concentration values of the oils samples can be predicted accurately and/or the samples are classified uniquely into classes. The prediction model and/or the identification of the classes is obtained when each sample is correlated to the characterisation information relating to said sample.

The multivariate statistical methods suitable for the present invention are for example represented by chemometric methods like principal component analysis (PCA), partial least squares regression (PLS), soft independent modelling of class analogy (SIMCA) and principal variables (PV).

Other multivariate statistical methods include: Principal component analysis¹⁴, principal component regression¹⁴, factor analysis², partial least squares¹⁴, fuzzy clustering¹⁶, artificial neural networks⁶, parallel factor analysis⁴, Tucker models¹³, generalized rank annihilation method⁹, Locally weighted regression¹⁵, ridge regression³, total least squares¹⁰, principal covariates regression⁷, Kohonen networks¹², linear or quadratic discriminant analysis¹¹, k-nearest neighbors based on rank-reduced distances¹, multilinear regression methods⁵, soft independent modeling of class analogies⁸, robustified versions of the above and/or obvious non-linear versions such as one obtained by allowing for interactions or crossproducts of variables, exponential transformations etc.

The term "describing the variation of the data variables" means that the latent variables retain the relevant information regarding the variation, whereas "noise" is preferably not giving any significant part in the latent variables.

### Other variables

The classification system may be obtained on the spectral information only. However, in some situations it may be appropriate to incorporate other variable(s) in the multivariate analyses.

These other variables may be variables relating to the lubricant oil sample supplying the spectral information or they may be variables that compensate for a specific condition of the lubricant oil sample.

Examples hereof may be the measurement of pH and temperature in the lubricant oil sample before subjecting it to spectroscopy. Thereby, variations in the other variables may be compensated for in the final prediction and/or classification.

In another embodiment other variables are variables relating to the lubricant oil sample to be characterized. Examples of these variables are oil type, origin, transport history, and process conditions.

### Pre-treatment

It is an advantage of the present invention that no pre-treatment of the lubricant oil sample is normally necessary.

However, for some of the samples it may be necessary to perform an adjustment before subjecting the sample to spectroscopy, as long as the lubricant oil samples being subjected to the method according to the invention have been directly obtained from an oil engine, a transmission, or any lubricant oil using unit without any changes in concentration, as defined in the appended claims.

Examples of pre-treatment may be adjustment of pH of the sample to a predetermined value, or heating or cooling the sample to a predetermined temperature. The sample may be treated with chemicals, e.g. in order to develop fluorescent complexes involving inherently non-fluorescent molecules in the sample.

Other types of pre-treatment include addition of chemical substances, measurement under a gradient imposed by varying additions of chemical substances, and simple chromatographic pre-treatments based on either chemical or physical separation principles.

### Prediction system

Another embodiment of the present invention is the prediction method for quantitative assessment of lubricant oil sample quality parameters.

The methods according to the invention provides means for predicting one or more lubricant oil quality parameters simultaneously and/or classifying the lubricant oil samples into at least two different classes correlated to the characterisation information, obtaining a trained prediction and/or classification system.

When the prediction system has been trained as discussed above, it is ready for prediction of one or more quality parameters lubricant oil samples with unknown characteristics.

The prediction system comprises the following components:
a) a sample domain for comprising the oil sample,
b) means for exposing the oil sample in the sample domain to electromagnetic radiation,
c) detecting means for recording at least one physical spectroscopic parameter of electromagnetic radiation transmitted and/or reflected and/or emitted from the sample,
d) computing means for performing data analysis of the at least one physical spectroscopic parameter, obtaining at least one data variable,
e) storage means for storing the at least one obtained data variable,
f) storage means for storing latent variables, multivariate prediction models and characterisation information of a trained prediction system,
g) processing means for providing at least one latent variable from the at least one obtained data variable of the sample,
h) means for correlating latent data variables from the sample with latent variables of the trained system, and
i) means for displaying the prediction(s) of a sample.

### Classification system

Another embodiment of the present invention is the classification for characterising a lubricant oil sample into at least one predetermined class. When the classification system has been trained as discussed above, it is ready for classifying lubricant oil samples with unknown characteristics. The classification system preferably comprises the following components:
a)-e) Identical to the steps for Prediction system,
f) storage means for storing latent variables, classification models and characterisation information of a trained classification system,
g) processing means for providing latent variables from data variables of the sample,
h) means for correlating latent data variables from the sample with latent variables of the trained system, and
i) means for displaying the characterisation class(es) of a sample.

For both the prediction and the classification system the following holds:
The sample domain may be a sample chamber for accommodating a container with a liquid, a solid or a semi-solid sample. However, the sample domain may also be a domain in a process stream, vessel or engine.

The lubricant oil sample may be obtained from an oil engine, a transmission, or any other lubricant oil containing and/or using unit.

The prediction and/or classification system may be provided as a whole unit, wherein the spectroscopy of the lubricant oil sample is conducted by the same unit from where the data relating to the characterisation classes of the lubricant oil sample is displayed.

It is however contemplated within the scope of the present invention, that the system is comprised of at least two units, wherein one unit is performing the steps a) to e), and another unit is performing the steps f) to i). Other units comprising other parts of the system are also contemplated, such as one unit performing the steps a) to f) or a) to g), and the other unit performing the rest of the steps. Yet another unit may perform steps a) to c) and the remaining steps may be performed in another unit.

By the system thus divided into two units, it is possible to obtain the spectroscopic information from a wide variety of decentralized locations and perform the processing centrally. The data or the prediction and/or classification system may then be transmitted by any suitable means, such as conventional data transmission lines, telephone lines, or via internet or intranet connections.

This configuration facilitates the use of the prediction and/or classification system since any process-engineer or technician may provide the lubricant oil sample and have it subjected to spectroscopic analyses obtaining data variables without the need of being capable of conducting the processing and correlating procedures at the sampling site. The engineer or technician may then obtain the prediction and classification results of the lubricant oil sample data from the central unit through a secured (internet) connection.

### Examples

### Example 1: Prediction of lubricant oil quality parameters by NIR

Thirty-nine samples of lubricant oil, a mix of new and used, were analyzed by near infrared (NIR) spectroscopy. By chemometric data analysis the spectra were correlated to laboratory values describing the oil quality.

The 39 oil samples were analyzed in a 1 mm cuvette on a NIRSystems 6500 (Foss, Hillerød, Denmark). NIR transmission spectra were recorded in the range from 1200 nm to 2500 nm. The same 39 samples were analyzed by an international approved laboratory for the following important oil quality parameters: Water concentration, Viscosity (at 40 and 100 degrees), Total Base number (TBN), Total Acid Number (TAN), Elemental analysis (e.g. metals) and particles. The NIR spectra were correlated to the laboratory values using chemometric modeling.

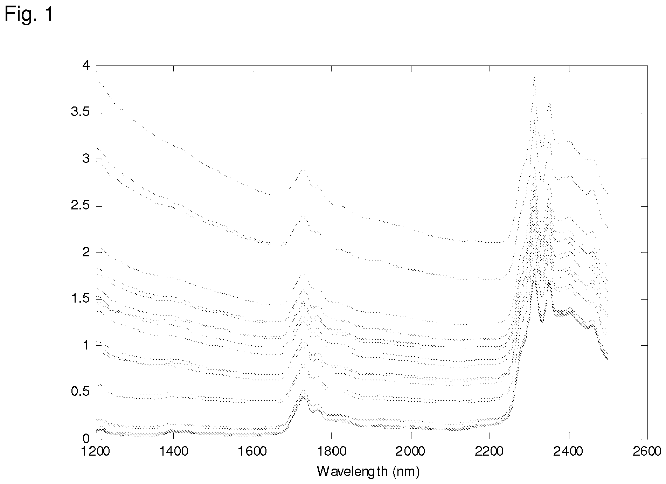

Figure 1 shows the measured NIR spectra. It is seen that there is a clear difference between the used oil samples (upper 17 curves) and fresh oil samples (lover curves).

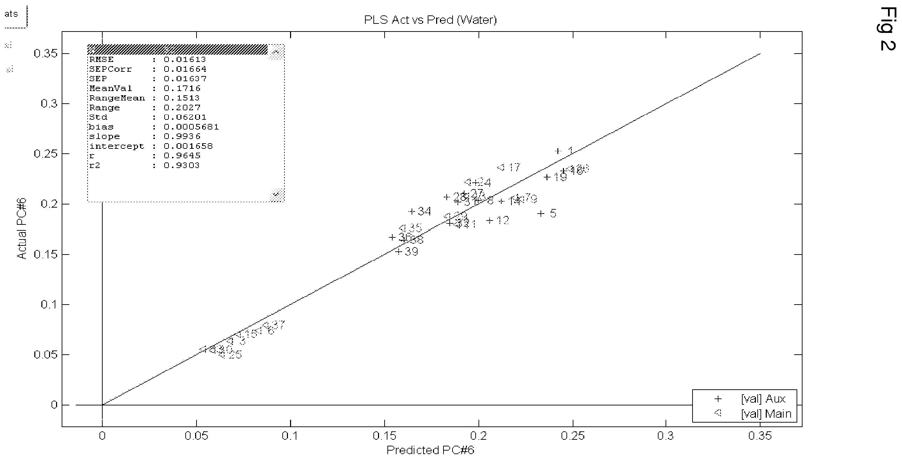

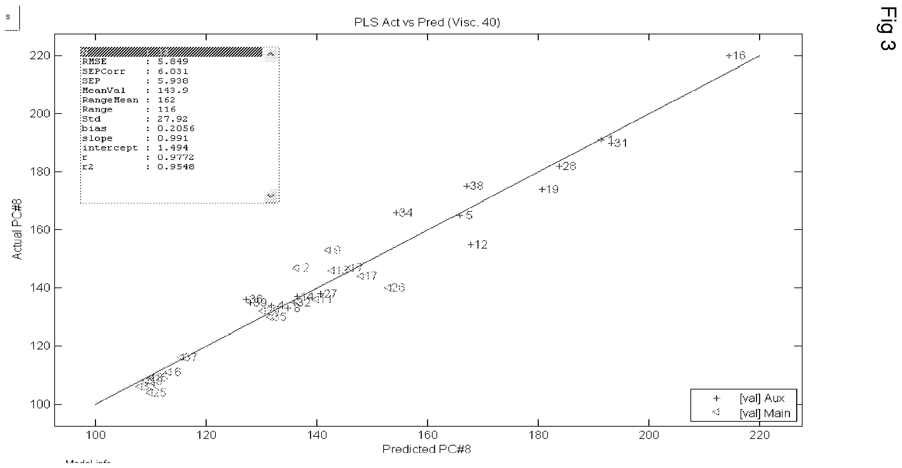

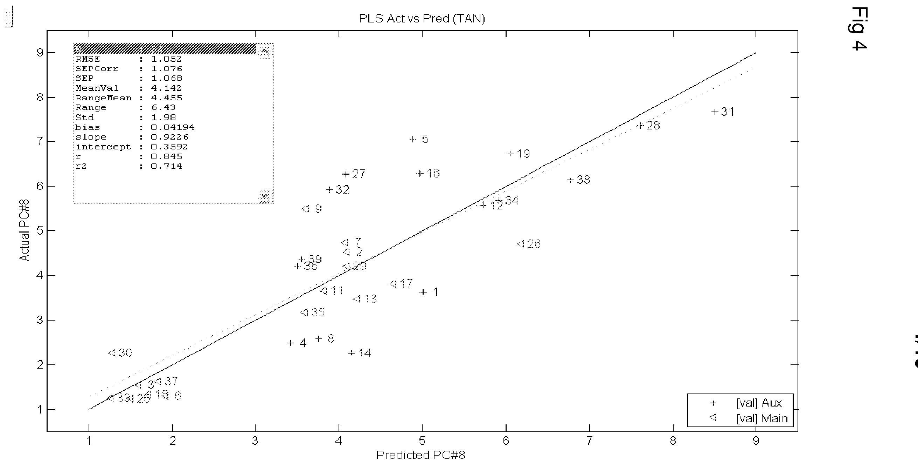

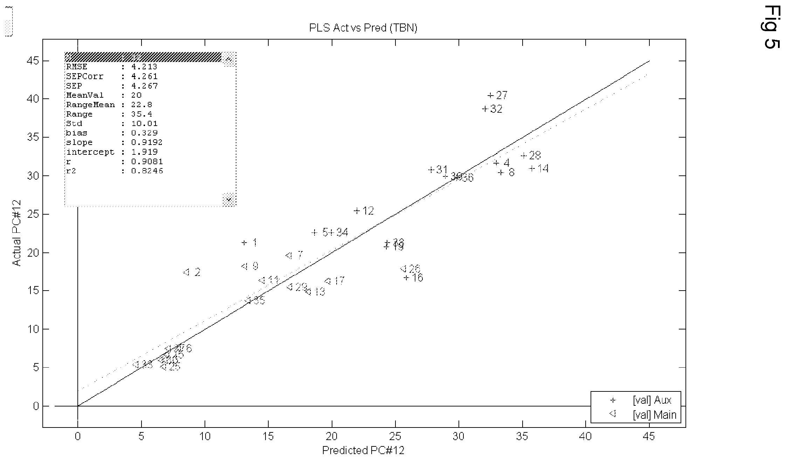

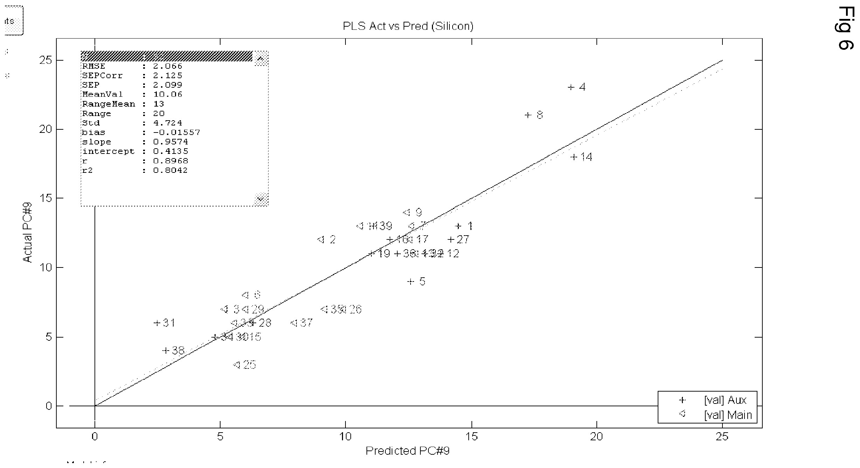

Figures 2 to 6 display plots showing the laboratory value versus the value predicted by the chemometric model and NIR spectroscop for water (Fig 2), viscosity at 40 degrees (Fig 3), TAN (Fig 4), TBN (Fig 5) and Silicon (Fig 6). Full cross validation is used to validate the models. From the actual versus predicted plots it is clearly observed that it is possible to predict different oil quality parameters by the present invention.

### Example 2: Classification of lubricant oil using fluorescence spectroscopy and chemometrics

Fifty-six lubricant oil samples, a mix of used and new oils from different engine types, were analyzed on a Fluoromax 3 Spectrograph. The samples were measured in a 2 mm quartz cuvette in front face geometry. 3D excitation/emission fluorescence landscapes were recorded on each sample with following settings: Excitation 230 nm - 400 nm, emission 250 nm - 600 nm.

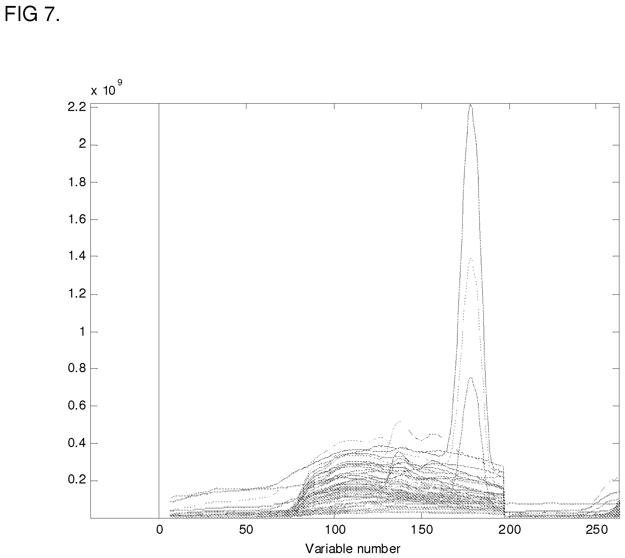

FIG 7 shows emission spectra at 230 nm excitation for all samples. It is seen that there are three samples with a specific pattern having a strong signal around 180nm. This verifies that this method also can be used for revealing strongly deviating samples e.g. according to special additives, oil types etc.

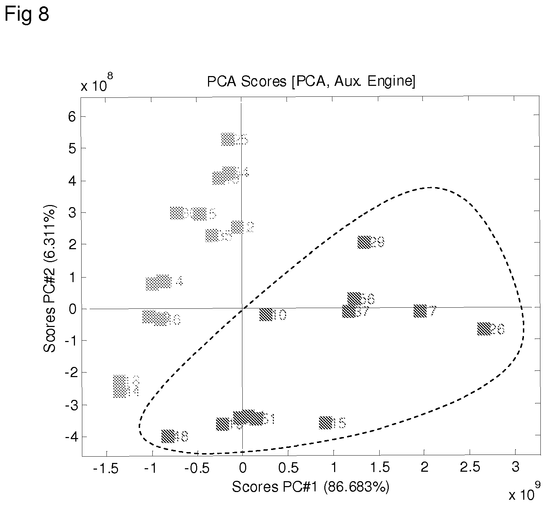

Fig 8.shows the obtained classification of new and used samples where the new samples are marked with the dotted circle.

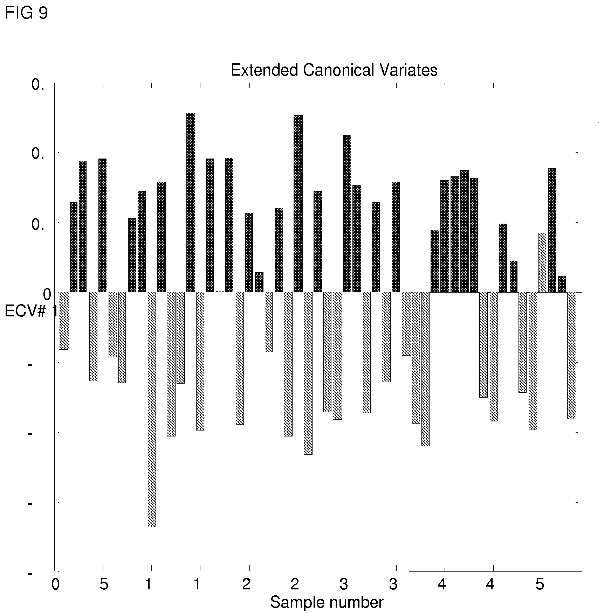

Fig 9 shows Extended Canonical Variates for the new samples (with negaive values) and old samples (with positive values)

### Example 3 Prediction of lubricant oil quality parameters by NIR

149 samples of lubricant oil from main engine vessels onboard ships, a mix of used and new, were analyzed by near inferred (NIR) spectroscopy. The spectra were then correlated to different laboratory values describing the oil quality using chemometric data analysis.

The 149 samples were analyzed on a MB3600 NIR FTIR spectrometer (Q-Interline). There were used 3 mm disposable cuvets and the samples were scanned from 1100 nm to 2500 nm measuring the transmission.

The same 149 samples was send to an international approved laboratory for analysis for oil quality parameters like: Viscosity, Density, Total Base number (TBN), Total Acid Number (TAN), Elemental analysis (e.g. metals, Sulfur and Phosphor).
The NIR spectra were correlated to the laboratory values using chemometric modeling by PLS.

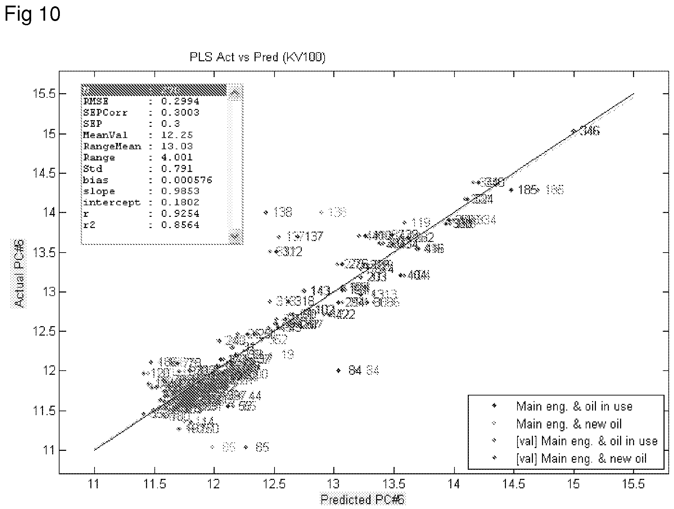

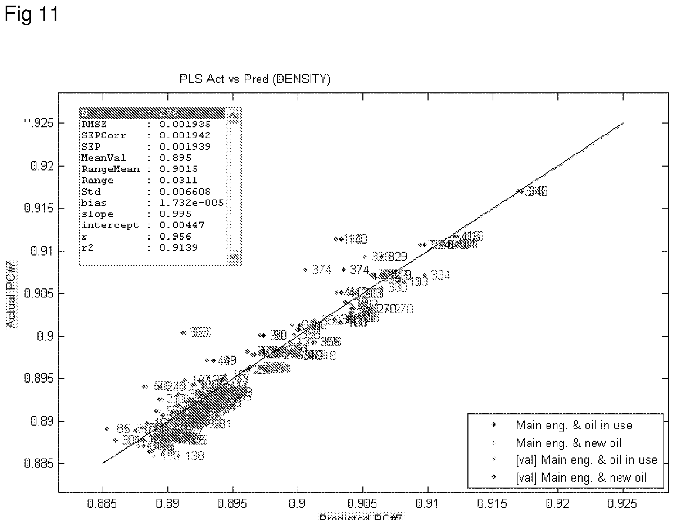

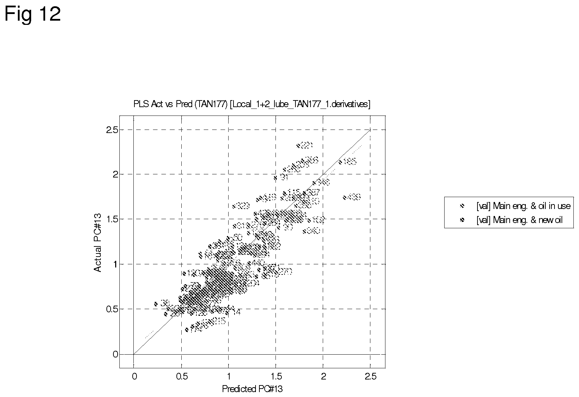

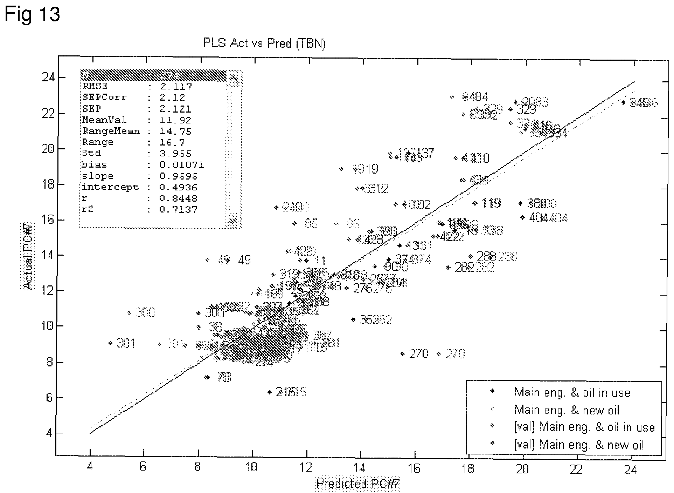

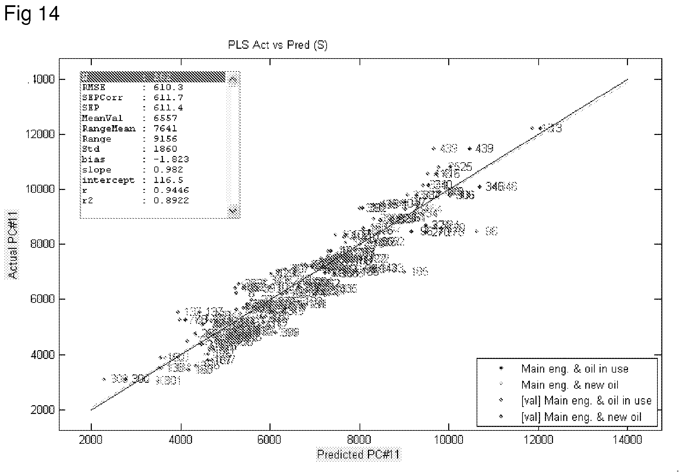

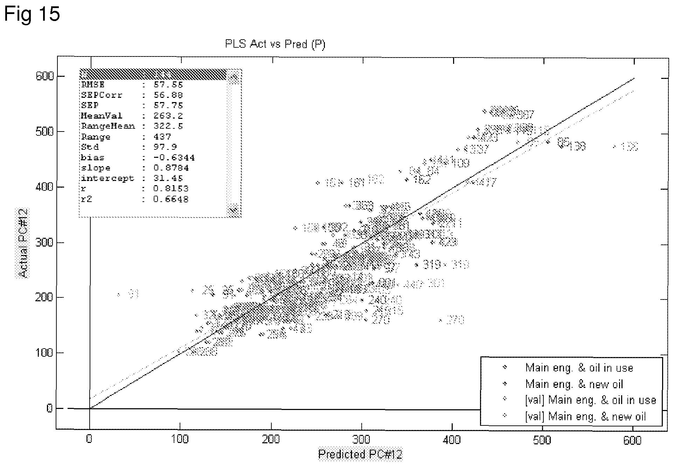

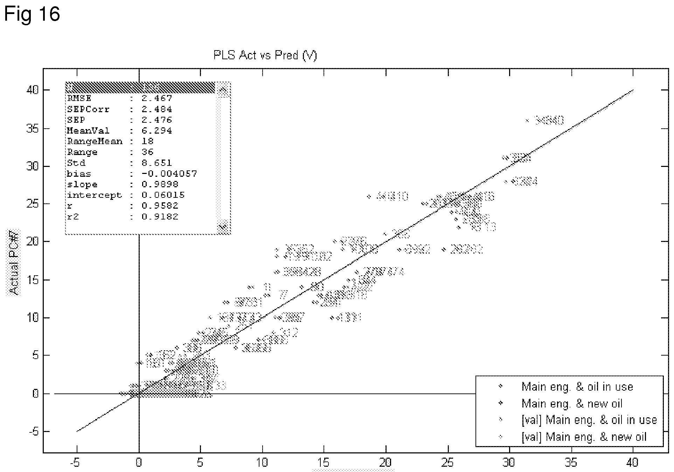

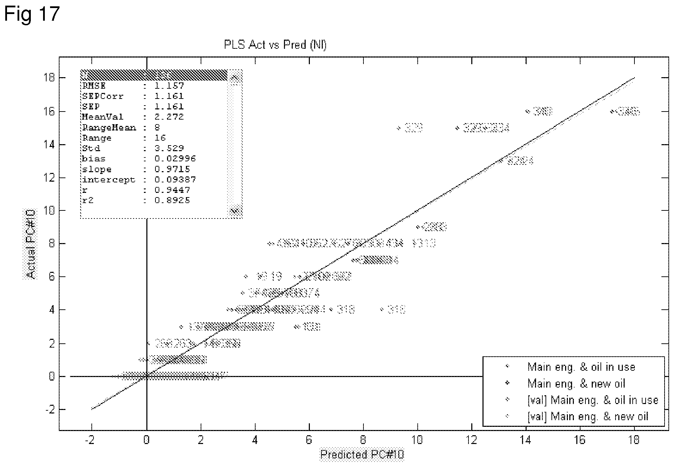

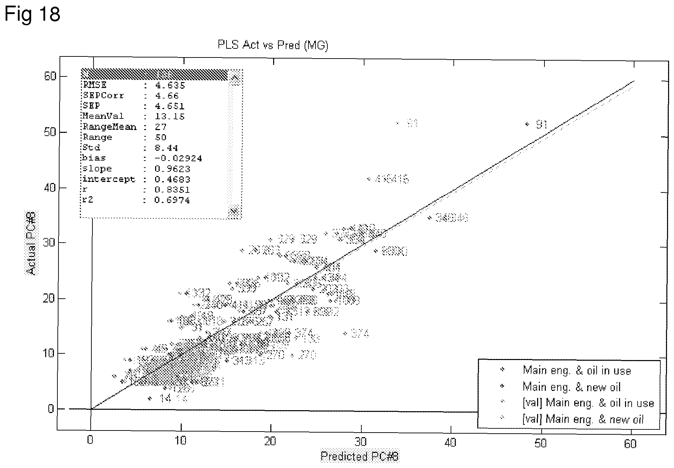

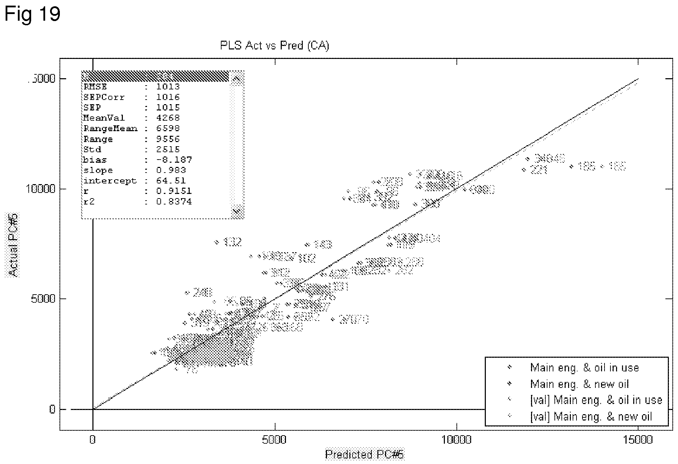

Figures 10 to 19 display plots showing the laboratory value versus the value predicted by the chemometric model and NIR spectroscop for viscosity at 40 degrees (Fig 10), density (Fig 11), TAN (Fig 12), TBN (Fig 13), sulfur (Fig 14), phosphor (Fig 15), vanadium (Fig 16), nickel (Fig 17), magnesium (Fig 18) and calcium (Fig 19). Full cross validation is used to validate the models. From the actual versus predicted plots it is clearly observed that it is possible to predict different oil quality parameters by the present invention.

It is clearly seen that it is possible to predict different oil quality parameters by the used method.

### Reference List

1. Alsberg BK, Goodacre R, Rowland JJ, Kell DB, Classification of pyrolysis mass spectra by fuzzy multivariate rule induction-comparison with regression, k-nearest neighbour, neural and decision-tree methods, Analytica Chimica Acta, 1997, 348, 389-407.
2. Bartholomew DJ, The foundation of factor analysis, Biometrika, 1984, 71, 221-232.
3. Björkström A, Sundberg R, A generalized view on continuum regression, Scandinavian Journal of Statistics, 1999, 26, 17-30.
4. Bro R, PARAFAC. Tutorial and applications, Chemom Intell Lab Syst, 1997, 38, 149-171.
5. Bro R, Multiway calibration. Multi-linear PLS, Journal of Chemometrics, 1996, 10, 47-61.
6. Cheng B, Titterington DM, Neural Networks: A Review from a Statistical Perspective, Statistical Science, 1994, 9, 2-54.
7. de Jong S, Kiers HAL, Principal covariates regression. Part 1. Theory, Chemom Intell Lab Syst, 1992, 14, 155-164.
8. Esbensen K, Wold S, SIMCA, MACUP, SELPLS, GDAM, SPACE & UNFOLD: The way towards regionalized principal components analysis and sub-constrained N-way decomposition - with geological illustrations, Proc Nord Symp Appl Statist, Stavanger, 1983,
9. Faber NM, Buydens LMC, Kateman G, Generalized rank annihilation method. I: derivation of eigenvalue problems, Journal of Chemometrics, 1994, 8, 147-154.
10. Golub GH, Hansen PC, O'leary D, Tikhonov Regularization and Total Least Squares, SIAM Journal of Numerical Analysis, 1999, 21, 185-194.
11. Indahl UG, Sahni NS, Kirkhus B, Næs T, Multivariate strategies for classification based on NIR-spectra - with application to mayonnaise, Chemom Intell Lab Syst, 1999, 49, 19-31.
12. Kohonen T, Self-organized formation of topologically correct feature maps, Biological Cybernetics, 1982, 43, 59-69.
13. Kruskal JB, Harshman RA, Lundy ME, Some relationships between Tucker's three-mode factor analysis and PARAFAC/CANDECOMP.1983,
14. Martens H, Næs T, Multivariate calibration. John Wiley & Sons, Chichester, 1989,
15. Næs T, Isaksson T, Some modifications of locally weighted regression (LWR), NIR news, 1994, 5, 8-9.
16. Rajko R, Treatment of model error in calibration by robust and fuzzy procedures, Analytical Letters, 1994, 27, 215-228.

## Claims

1. A method of training a system for characterising a lubricant oil sample, said lubricant oil sample being obtained directly from an oil engine, a transmission, or any other lubricant oil using unit and said lubricant oil sample being subjected to the method without any changes in concentration, said method comprising the steps of
a) providing a batch of lubricant oil samples having a predetermined content for training the system to recognise at least one characteristic of the sample,
b) exposing a lubricant oil sample from said batch to electromagnetic radiation within one or both range(s) of wavelengths from 200 nm to 800 nm and/or from 750 nm to 3000 nm,
c) monitoring the electromagnetic radiation emitted from the lubricant oil sample from said batch at an exposing range from 200 nm to 800 nm, and/or monitoring the
electromagnetic radiation transmitted through and/or reflected from the lubricant oil sample from said batch at an exposing range from 750 nm to 3000 nm, in order to determine at
least one physical spectroscopic parameter,
d) repeating step b) to c) until the at least one physical spectroscopic parameter of all lubricant oil samples of said batch have been determined,
e) performing data processing of the obtained physical spectroscopic parameters thereby obtaining data variables,
f) performing a multivariate data analysis of the obtained data variables obtaining model parameters describing the variation of the data variables,
g) obtaining characterisation information related to each lubricant oil sample from said batch.

2. The method according to claim 1, furthermore comprising the step of predicting one or more lubricant oil quality parameters simultaneously and/or classifying the lubricant oil samples into at least two different classes correlated to the characterisation information, obtaining a trained prediction and/or classification system.

3. The method according to any of the previous claims, wherein step g) further comprises selection of latent variables being weighted averages of data variables, and wherein the ratio of number of training samples to the expected number of latent variables is at least 5:1, preferably at least 10:1, more preferably 50:1, and even more preferred 100:1.

4. A method for characterising a lubricant oil sample, said lubricant oil sample being obtained directly from an oil engine, a transmission, or any other
lubricant oil using unit and said lubricant oil sample being subjected to the method without any changes in concentration, said method comprising the steps of
a) obtaining the lubricant oil sample,
b) exposing the lubricant oil sample to electromagnetic radiation within one or both range(s) of wavelengths from 200 nm to 800 nm and/or from 750 nm to 3000 nm,
c) determining at least one physical spectroscopic parameter of electromagnetic radiation emitted from the lubricant oil sample at an exposing range from 200 nm to 800 nm, and/or determining at least one physical spectroscopic parameter of electromagnetic radiation transmitted and/or reflected from the lubricant oil sample at an exposing range from 750 nm to 3000 nm,
d) performing data processing of the obtained at least one physical spectroscopic parameter obtaining at least one data variable,
e) storing the obtained at least one data variable,
f) providing at least one latent variable from data variables of the sample,
g) obtaining at least one latent variable from a trained prediction and/or classification system,
h) correlating latent data variables from the sample with latent variables of the trained prediction and classification system, to obtain quantitative predictions and/or quality predictions and/or at least one characterisation class of the sample, and
i) displaying the quantitative predictions and/or quality predictions and/or at least one characterisation class of the sample.

5. The method according to any of the previous claims, wherein the physical spectroscopic parameter determined is selected from the group of fluorescence intensity, fluorescence lifetime, phosphorescence intensity, phosphorescence lifetime, depolarisation, quantum yield, phase-resolved emission, and circular depolarization.

6. The method according to any of the previous claims, wherein the physical
spectroscopic parameter to be determined in order to perform a data analysis are the absorbance and/or transmission and/or reflectance values as a function of wavelength at the exposing range from 750 nm to 3000 nm.

7. The method according to any of the previous claims, wherein the data subsequently being processed by multivariate data analysis is selected from the group of: Principal component analysis, principal component regression, factor analysis, partial least squares, fuzzy clustering, artificial neural networks, parallel factor analysis, Tucker models, generalised rank annihilation method, Locally weighted regression, ridge regression, total least squares, principal covariates regression, Kohonen networks, linear or quadratic discriminant analysis, k-nearest neighbours based on rank-reduced distances, multilinear regression methods, soft independent modelling of class analogies, robustified versions of the above and/or obvious non-linear versions such as one obtained by allowing for interactions or cross products of variables, and exponential transformations.

8. The method according to any of the previous claims, wherein the data analysis of step f) in the method of claim 1 and step d) in the method of claim 4 is performed on the spectral information being either a one-way matrix of spectral information, a two-way matrix of spectral information, a three-way matrix of spectral information, a four-way matrix of spectral information and, a five-way or higher order matrix of spectral information or mixtures thereof, and at least one other variable related to the lubricant oil sample is selected from a pH value of the lubricant oil sample, concentration of any other relevant compound in the lubricant oil sample, temperature or any other physical property of the lubricant oil sample or property of the lubricant oil sample or the place from which the lubricant oil sample was taken.

9. The method according to any of claims 2-8, wherein the lubricant oil quality parameter relates to at least one of the group of the Water Concentration, Total Base Number (TBN), Total Acid Number (TAN), Viscosity (at different temperatures) and the content of Particles, Silicon, Sodium, Boron, Iron, Aluminum, Chromium, Molybdenum, Copper, Lead, Tin, Nickel, Titanium, Silver, Phosphorus, Zinc, Calcium, Barium, Magnesium, or Sulfur.

10. The method according to any of the previous claims, wherein the characterising of a lubricant oil sample takes place at-line or on-line.

## Patentansprüche

1. Verfahren zum Trainieren eines Systems zum Charakterisieren einer Schmierölprobe, wobei die Schmierölprobe direkt von einem Ölmotor, einem Getriebe oder einer beliebigen anderen Schmieröl verwendenden Einheit erhalten wird und die Schmierölprobe dem Verfahren ohne jegliche Änderungen der Konzentration ausgesetzt wird, wobei das Verfahren die folgenden Schritte umfasst
a) Bereitstellen eines Batches an Schmierölproben, die einen zuvor festgelegten Inhalt aufweisen, um das System zu trainieren, zumindest eine Charakteristik der Probe zu erkennen,
b) Exponieren einer Schmierölprobe aus dem Batch gegenüber elektromagnetischer Strahlung innerhalb eines oder beider Bereiche von Wellenlängen von 200 nm bis 800 nm und/oder von 750 nm bis 3000 nm,
c) Überwachen der elektromagnetischen Strahlung, die von der Schmierölprobe aus dem Batch in einem Expositionsbereich von 200 nm bis 800 nm emittiert wird, und/oder Überwachen der elektromagnetischen Strahlung, die durch die Schmierölprobe aus dem Batch in einem Expositionsbereich von 750 nm bis 3000 nm übertragen und/oder davon reflektiert wird, um zumindest einen physischen spektroskopischen Parameter zu bestimmen,
d) Wiederholen von Schritt b) bis c), bis der zumindest eine physische spektroskopische Parameter aller Schmierölproben des Batches bestimmt worden ist,
e) Durchführen von Datenverarbeitung der erhaltenen physischen spektroskopischen Parameter, wodurch Datenvariablen erhalten werden,
f) Durchführen einer multivariaten Datenanalyse der erhaltenen Datenvariablen, wodurch Modellparameter erhalten werden, die die Variation der Datenvariablen beschreiben,
g) Erhalten von Charakterisierungsinformationen in Bezug auf jede Schmierölprobe aus dem Batch.

2. Verfahren nach Anspruch 1, ferner umfassend den Schritt des gleichzeitigen Vorhersagens von einem oder mehreren Schmierölqualitätsparametern und/oder des Klassifizierens der Schmierölproben in zumindest zwei unterschiedliche Klassen korreliert mit den Charakterisierungsinformationen, wodurch ein trainiertes Vorhersage- und/oder Klassifizierungssystem erhalten wird.

3. Verfahren nach einem der vorhergehenden Ansprüche, wobei Schritt g) ferner eine Auswahl von latenten Variablen umfasst, bei denen es sich um gewichtete Durchschnitte von Datenvariablen handelt, und wobei das Verhältnis der Anzahl an Trainingsproben zu der erwarteten Anzahl an latenten Variablen zumindest 5:1 beträgt, bevorzugt zumindest 10:1, bevorzugter 50:1 und sogar noch bevorzugter 100:1.

4. Verfahren zum Charakterisieren einer Schmierölprobe, wobei die Schmierölprobe direkt von einem Ölmotor, einem Getriebe oder einer beliebigen anderen Schmieröl verwendenden Einheit erhalten wird und die Schmierölprobe dem Verfahren ohne jegliche Änderungen der Konzentration ausgesetzt wird, wobei das Verfahren die folgenden Schritte umfasst
a) Erhalten der Schmierölprobe,
b) Exponieren der Schmierölprobe gegenüber elektromagnetischer Strahlung innerhalb eines oder beider Bereiche von Wellenlängen von 200 nm bis 800 nm und/oder von 750 nm bis 3000 nm,
c) Bestimmen von zumindest einem physischen spektroskopischen Parameter von elektromagnetischer Strahlung, die von der Schmierölprobe in einem Expositionsbereich von 200 nm bis 800 nm emittiert wird, und/oder
Bestimmen von zumindest einem physischen spektroskopischen Parameter von elektromagnetischer Strahlung, die von der Schmierölprobe in einem Expositionsbereich von 750 nm bis 3000 nm übertragen und/oder reflektiert wird,
d) Durchführen von Datenverarbeitung des erhaltenen zumindest einen physischen spektroskopischen Parameters, wodurch zumindest eine Datenvariable erhalten wird,
e) Speichern der erhaltenen zumindest einen Datenvariable,
f) Bereitstellen von zumindest einer latenten Variable von Datenvariablen der Probe,
g) Erhalten von zumindest einer latenten Variable von einem trainierten Vorhersage- und/oder Klassifizierungssystem,
h) Korrelieren von latenten Datenvariablen aus der Probe mit latenten Variablen des trainierten Vorhersage- und Klassifizierungssystems, um quantitative Vorhersagen und/oder Qualitätsvorhersagen und/oder zumindest eine Charakterisierungsklasse der Probe zu erhalten, und
i) Anzeigen der quantitativen Vorhersagen und/oder Qualitätsvorhersagen und/oder zumindest eine Charakterisierungsklasse der Probe.

5. Verfahren nach einem der vorhergehenden Ansprüche, wobei der bestimmte physische spektroskopische Parameter aus der Gruppe von Fluoreszenzintensität, Fluoreszenzlebensdauer, Phosphoreszenzintensität, Phosphoreszenzlebensdauer, Depolarisation, Quantenausbeute, phasenaufgelöster Emission und zirkulärer Depolarisation ausgewählt ist.

6. Verfahren nach einem der vorhergehenden Ansprüche, wobei der physische spektroskopische Parameter, der zu bestimmen ist, um eine Datenanalyse durchzuführen, die Absorptions- und/oder Übertragungs- und/oder Reflexionswerte in Abhängigkeit von der Wellenlänge im Expositionsbereich von 750 nm bis 3000 nm sind.

7. Verfahren nach einem der vorhergehenden Ansprüche, wobei die Daten, die anschließend durch multivariate Datenanalyse verarbeitet werden, aus der Gruppe des Folgenden ausgewählt sind: Hauptkomponentenanalyse, Hauptkomponentenregression, Faktorenanalyse, partielle kleinste Quadrate, Fuzzy-Clustering, künstliche neuronale Netzwerke, parallele Faktorenanalyse, Tucker-Modelle, Verfahren der generalisierten Rangauslöschung, lokal gewichtete Regression, Ridge-Regression, Methode der kleinsten Quadrate, Hauptkovariablenregression, Kohonen-Netzwerke, lineare oder quadratische Diskriminantenanalyse, k-nächste Nachbarn auf der Grundlage von rangreduzierten Abständen, Verfahren der multilinearen Regression, weiche unabhängige Modellierung von Klassenanalogien, verfestigte Versionen des Vorstehenden und/oder eindeutige nichtlineare Versionen wie zum Beispiel eine, die erhalten wird, indem Interaktionen oder Kreuzprodukte von Variablen zugelassen werden, und exponentielle Transformationen.

8. Verfahren nach einem der vorhergehenden Ansprüche, wobei die Datenanalyse aus Schritt f) in dem Verfahren nach Anspruch 1 und Schritt d) in dem Verfahren nach Anspruch 4 an den Spektralinformationen durchgeführt wird, die entweder eine Einwegmatrix an Spektralinformationen, eine Zweiwegmatrix an Spektralinformationen, eine Dreiwegmatrix an Spektralinformationen, eine Vierwegmatrix an Spektralinformationen und eine Fünfweg- oder höhere Matrix an Spektralinformationen oder Mischungen F davon sind, und zumindest eine andere Variable ist ausgewählt, die sich auf die Schmierölprobe bezieht, aus einem pH-Wert der Schmierölprobe, einer Konzentration einer beliebigen anderen relevanten Verbindung in der Schmierölprobe, einer Temperatur oder einer beliebigen anderen physischen Eigenschaft der Schmierölprobe oder einer Eigenschaft der Schmierölprobe oder der Stelle, von der die Schmierölprobe entnommen wurde.

9. Verfahren nach einem der Ansprüche 2-8, wobei sich der Schmierölqualitätsparameter auf zumindest eines aus der Gruppe der Wasserkonzentration, der Gesamtbasenzahl (TBN), der Gesamtsäurezahl (TAN), der Viskosität (bei unterschiedlichen Temperaturen) und des Gehalts von Partikeln, Silizium, Natrium, Bor, Eisen, Aluminium, Chrom, Molybdän, Kupfer, Blei, Zinn, Nickel, Titan, Silber, Phosphor, Zink, Calcium, Barium, Magnesium oder Schwefel, bezieht.

10. Verfahren nach einem der vorhergehenden Ansprüche, wobei das Charakterisieren einer Schmierölprobe At-Line oder On-Line stattfindet.

## Revendications

1. Procédé d'apprentissage d'un système pour caractériser un échantillon d'huile lubrifiante, ledit échantillon d'huile lubrifiante étant obtenu directement à partir d'un moteur à huile, d'une transmission, ou de toute autre unité utilisant une huile lubrifiante et ledit échantillon d'huile lubrifiante étant soumis au procédé sans aucun changement de concentration, ledit procédé comprenant les étapes de
a) fourniture d'un lot d'échantillons d'huile lubrifiante ayant un contenu prédéterminé pour entraîner le système à reconnaître au moins une caractéristique de l'échantillon,
b) exposition d'un échantillon d'huile lubrifiante provenant dudit lot à un rayonnement électromagnétique dans l'une ou les deux plages de longueurs d'onde allant de 200 nm à 800 nm et/ou de 750 nm à 3 000 nm,
c) la surveillance du rayonnement électromagnétique émis par l'échantillon d'huile lubrifiante provenant dudit lot à une plage d'exposition allant de 200 nm à 800 nm, et/ou la surveillance du rayonnement électromagnétique transmis à travers et/ou réfléchi par l'échantillon d'huile lubrifiante provenant dudit lot à une plage d'exposition allant de 750 nm à 3 000 nm, afin de déterminer au moins un paramètre spectroscopique physique
d) répétition de l'étape b) à c) jusqu'à ce que l'au moins un paramètre spectroscopique physique de tous les échantillons d'huile lubrifiante dudit lot ait été déterminé,
e) mise en œuvre d'un traitement de données des paramètres spectroscopiques physiques obtenus pour ainsi obtenir des variables de données,
f) mise en œuvre d'une analyse de données à plusieurs variables des variables de données obtenues pour obtenir des paramètres de modèle décrivant la variation des variables de données,
g) obtention d'informations de caractérisation relatives à chaque échantillon d'huile lubrifiante provenant dudit lot.

2. Procédé selon la revendication 1, comprenant en outre l'étape de prédiction simultanée d'un ou de plusieurs paramètres de qualité d'huile lubrifiante et/ou de classement des échantillons d'huile lubrifiante en au moins deux classes différentes corrélées aux informations de caractérisation, pour obtenir un système entraîné de prédiction et/ou de classement.

3. Procédé selon l'une quelconque des revendications précédentes, dans lequel l'étape g) comprend en outre la sélection de variables latentes qui sont des moyennes pondérées de variables de données, et dans lequel le rapport entre le nombre d'échantillons d'entraînement et le nombre attendu de variables latentes est au moins de 5 : 1, de préférence au moins de 10 : 1, de manière davantage préférée de 50 : 1, et de manière encore plus préférée de 100 : 1.

4. Procédé de caractérisation d'un échantillon d'huile lubrifiante, ledit échantillon d'huile lubrifiante étant obtenu directement à partir d'un moteur à huile, d'une transmission, ou de toute autre unité utilisant une huile lubrifiante et ledit échantillon d'huile lubrifiante étant soumis au procédé sans aucun changement de concentration, ledit procédé comprenant les étapes de
a) obtention de l'échantillon d'huile lubrifiante,
b) exposition de l'échantillon d'huile lubrifiante à un rayonnement électromagnétique dans l'une ou les deux plages de longueurs d'onde allant de 200 nm à 800 nm et/ou de 750 nm à 3 000 nm,
c) détermination d'au moins un paramètre spectroscopique physique de rayonnement électromagnétique émis par l'échantillon d'huile lubrifiante à une plage d'exposition allant de 200 nm à 800 nm, et/ou la détermination d'au moins un paramètre spectroscopique physique de rayonnement électromagnétique transmis et/ou réfléchi par l'échantillon d'huile lubrifiante à une plage d'exposition allant de 750 nm à 3 000 nm,
d) mise en œuvre d'un traitement de données de l'au moins un paramètre spectroscopique physique obtenu pour obtenir au moins une variable de données,
e) stockage de l'au moins une variable de données obtenue,
f) fourniture d'au moins une variable latente provenant des variables de données de l'échantillon,
g) obtention d'au moins une variable latente à partir d'un système entraîné de prédiction et/ou de classement,
h) mise en corrélation des variables de données latentes provenant de l'échantillon avec des variables latentes du système entraîné de prédiction et de classement, pour obtenir des prédictions quantitatives et/ou des prédictions qualitatives et/ou au moins une classe de caractérisation de l'échantillon, et
i) affichage des prédictions quantitatives et/ou des prédictions qualitatives et/ou d'au moins une classe de caractérisation de l'échantillon.

5. Procédé selon l'une quelconque des revendications précédentes, dans lequel le paramètre spectroscopique physique déterminé est sélectionné dans le groupe comprenant une intensité de fluorescence, une durée de vie de fluorescence, une intensité de phosphorescence, une durée de vie de phosphorescence, une dépolarisation, un rendement quantique, une émission résolue en phase, et une dépolarisation circulaire.

6. Procédé selon l'une quelconque des revendications précédentes, dans lequel le paramètre spectroscopique physique à déterminer afin de mettre en œuvre une analyse de données est les valeurs d'absorbance et/ou de transmission et/ou de facteur de réflexion en fonction de la longueur d'onde dans la plage d'exposition allant de 750 nm à 3 000 nm.

7. Procédé selon l'une quelconque des revendications précédentes, dans lequel les données traitées par la suite par une analyse de données à plusieurs variables sont sélectionnées dans le groupe comprenant : une analyse en composantes principales, une régression en composantes principales, une analyse factorielle, les moindres carrés partiels, une agrégation floue, les réseaux de neurones artificiels, une analyse factorielle parallèle, les modèles de Tucker, une méthode d'annihilation généralisée de rangs, une régression localement pondérée, une régression ridge, les moindres carrés totaux, une régression en covariables principales, les réseaux de Kohonen, une analyse discriminante linéaire ou quadratique, une méthode des k plus proches voisins basée sur des distances réduites en rangs, les méthodes de régression multilinéaire, une modélisation indépendante douce d'analogies de classe, les versions robustifiées des versions non linéaires ci-dessus et/ou les versions évidentes telles que celle obtenue en permettant des interactions ou des produits croisés de variables, et des transformations exponentielles.

8. Procédé selon l'une quelconque des revendications précédentes, dans lequel l'analyse de données dans l'étape f) dans le procédé selon la revendication 1 et l'étape d) dans le procédé selon la revendication 4 est effectuée sur les informations spectrales qui est l'une ou l'autre parmi une matrice unidirectionnelle d'informations spectrales, une matrice bidirectionnelle d'informations spectrales, une matrice tridirectionnelle d'informations spectrales, une matrice quadridirectionnelle d'informations spectrales et une matrice pentadirectionnelle ou d'ordre supérieur d'informations spectrales ou les mélanges de celles-ci, et au moins une autre variable relative à l'échantillon d'huile lubrifiante est sélectionnée parmi une valeur de pH de l'échantillon d'huile lubrifiante, une concentration de tout autre composé d'intérêt dans l'échantillon d'huile lubrifiante, une température ou toute autre propriété physique de l'échantillon d'huile lubrifiante ou une propriété de l'échantillon d'huile lubrifiante ou l'endroit où a été prélevé l'échantillon d'huile lubrifiante.

9. Procédé selon l'une quelconque des revendications 2 à 8, dans lequel le paramètre de qualité d'huile lubrifiante concerne au moins l'un du groupe consistué par la concentration en eau, l'indice de base total (TBN), l'indice d'acide total (TAN), la viscosité (à différentes températures) et la teneur en particules, silicium, sodium, bore, fer, aluminium, chrome, molybdène, cuivre, plomb, étain, nickel, titane, argent, phosphore, zinc, calcium, baryum, magnésium, ou soufre.

10. Procédé selon l'une quelconque des revendications précédentes, dans lequel la caractérisation d'un échantillon d'huile lubrifiante a lieu au niveau de la ligne ou en ligne.
